# FASCICULE DE BREVET EUROPEEN

(11) **EP 1 834 195 B1**
(45) Date de publication et mention de la délivrance du brevet: **17.03.2010**
(21) Numéro de dépôt: 06709044.9
(22) Date de dépôt: 06.01.2006
(51) Int. Cl.: G02B 1/04, G02B 5/23, A61L 27/16, C07D 311/92

(54) **LENTILLE INTRAOCULAIRE PHOTOCHROMIQUE**
FOTOCHROME INTRAOKULARLINSE
PHOTOCHROMIC INTRAOCULAR LENS

(30) Priorité: 07.01.2005 FR 0500199
(43) Date de publication de la demande: 19.09.2007
(73) Titulaire: Ioltechnologie-Production, 17180 Perigny (FR)
(72) Inventeur: BERNARD, Pascal, F-17137 Nieul sur Mer (FR); DOLATKHANI, Marc, F-33610 Cestas (FR); PAGNOUX, Anne, F-33114 Le Barp (FR); HUPIN, Christophe, F-33770 Salles (FR)
(74) Mandataire: Caen, Thierry Alain
(86) Numéro de dépôt international: PCT/FR2006/000029
(87) Numéro de publication internationale: WO 2006/072748

(56) Documents cités:
- US-A- 6 113 814
- US-B1- 6 224 210
- PAVEL P. ZACK, MICHAIL A. OSTROVSKY, V. TSEHOMSKY, AND JU. ZAIKIN: "UV-blue-light-absorbing photochromic intraocular lens for protection against age-related macular degeneration development" SPIE PROCEEDINGS, vol. 3579, 20 mars 2003 (2003-03-20), pages 102-104, XP002341284

## Description

La présente invention concerne une lentille intraoculaire capsulaire souple ou rigide, hydrophile ou hydrophobe, constituée, au moins en partie, d'un copolymère présentant des unités monomères photochromiques de sorte que l'implant, ou une partie de celui-ci change de couleur réversiblement lorsqu'il est exposé à la lumière, par exemple aux rayons ultraviolets.

Le cristallin est une lentille contenue dans un sac appelé sac capsulaire, situé à l'arrière de l'iris. La cataracte consiste en une opacification du cristallin.

Une opération de la cataracte connue, consiste à découper une grande partie de la paroi antérieure appelée capsule antérieure du sac capsulaire et à en extraire le cristallin. On remplace alors, dans le sac capsulaire, le cristallin extrait, par une lentille intraoculaire.

Les lentilles intraoculaires peuvent se présenter sous forme d'implant dit "rigide" ou d'implant dit "souple".

Les implants dits "souple" présentent l'avantage d'être plus facile à positionner et à stabiliser dans le sac capsulaire que les implants rigides.

Les implants souples sont généralement constitués d'une partie optique centrale formant la lentille et d'une partie haptique comprenant un ou plusieurs haptiques, situés à la périphérie de l'élément optique. Ces éléments haptiques peuvent être annulaire, plats ou sous forme d'anses.

On a déjà envisagé d'utiliser des matériaux colorés pour la fabrication de lentilles intraoculaires et ce en vue de remédier à certains troubles transitoires ou permanents de la vision.

De tels troubles ont notamment été décrits par H. R TAYLOR et al (Department of Opthalmology, Royal and Victorian Hospital Melbourne), qui ont montrés l'implication d'une exposition de la rétine à une couleur bleue intense (longueur d'ondes comprise entre 450 et 500 mm) dans le ptérygion et la kératopathie en gouttelettes.

Les lentilles intraoculaires colorées ont pour effet d'absorber certaines plages de longueur d'ondes, notamment les ultra-violets ci-après « UV ») et les bleus.

De telles lentilles intraoculaires colorées sont par exemple décrites dans la demande de brevet français n° 0215454 déposée le 6 décembre 2002 par la société IOLTECHNOLOGIE-PRODUCTION.

La demande de brevet EP-A-589 809 décrit des lentilles intraoculaires dont la coloration est réalisée par l'utilisation d'un adjuvant de transmission lumineuse, à savoir un colorant biocompatible avec le milieu interne de l'oeil humain.

Le brevet US-A-5662707 décrit des lentilles intraoculaires dont la coloration est réalisée par l'utilisation d'une poudre polymérisable introduite pendant la constitution du polymère utilisé dans la fabrication de la lentille intraoculaire.

Le brevet US 6 224 210 décrit des lentilles oculaires artificielles multifocales constituées d'un matériau photochromique qui permet de faire varier la transparence de la lentille de manière inversement proportionnelle à l'éclairement.

Cependant, ces lentilles intraoculaires présentent l'inconvénient de diminuer la perception visuelle lorsque la luminosité baisse. Ainsi, le passage d'une zone lumineuse à une zone obscure, créée une gène pour le porteur de la lentille intraoculaire.

Les inventeurs ont donc cherché à mettre au point des lentilles intraoculaires qui permettent de protéger la rétine de l'agression des rayons lumineux, notamment les bleus (longueurs d'onde de 450 à 500 nm) et les rayons UV (longueurs d'onde comprises entre 200 et 380 nm) en obviant les inconvénients des lentilles connues.

De telles lentilles doivent en outre être pharmaceutiquement acceptables, c'est-à-dire constituées de matériaux acceptables par l'organisme et ne relargant pas de composés toxiques ou agressifs vis-à-vis des tissus.

Ainsi, l'invention consiste en une lentille intraoculaire caractérisée en ce qu'elle comporte au moins un polymère photochromique pharmaceutiquement acceptable permettant à ladite lentille intraoculaire, en tout ou partie, de changer de couleur réversiblement lorsqu'elle est exposée à la lumière.

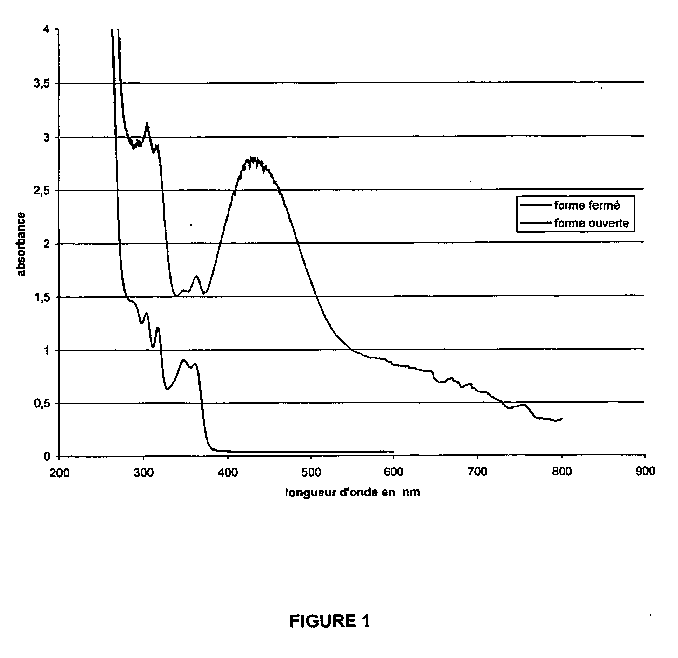

La figure 1 représente le spectre d'absorbance du monomère photochrome méthacrylique, un polymère photochromique préféré utilisable selon l'invention.

Les composés photochromiques sont des matériaux bien connus pour changer de couleur lorsqu'ils sont exposés à la lumière, notamment aux rayons ultraviolets.

Les inventeurs ont privilégié les composés photochromiques qui changent de couleur de manière réversible lorsqu'ils sont exposés à la lumière, notamment aux rayons ultraviolets.

De tels composés sont connus en tant que tels et utilisés dans de nombreuses applications, notamment dans le domaine optique où ils ont été mis en oeuvre pour brunir des verres de lunettes au soleil ou changer la couleur des lentilles de contact.

Une lentille intraoculaire selon l'invention peut comporter une partie optique et une partie haptique constituée d'un ou plusieurs éléments, la partie optique au moins comportant un polymère photochromique pharmaceutiquement acceptable, de sorte que la lentille intraoculaire ou au moins sa partie optique peut changer de couleur de manière réversible

Ledit polymère comprend généralement au moins un monomère photochromique et au moins un autre monomère, non photochromique.

Le monomère photochromique peut être, notamment, de type spiro-indolino-spiranes, spirobenzothiazolo-benzopyranes, spriroindolino-benzothiopyranes, spiro-indolino-oxazines ou naphtopyranes.

Des monomères photochromiques convenant tout particulièrement pour la réalisation de lentilles intraoculaires selon la présente invention, ont été décrits dans la demande de brevet français n°0405516, déposée le 24 mai

L'invention concerne une lentille intraoculaire comportant au moins un polymère photochromique pharmaceutiquement acceptable permettant à ladite lentille intraoculaire, en tout ou partie, de changer de couleur réversiblement lorsqu'elle est exposée à la lumière, ledit polymère photochromique étant formé d'au moins un monomère photochromique qui est un composé du type 3,3-diaryl-3H-naphto[2,1-b]pyrane substitué polymérisable de formule (I) : 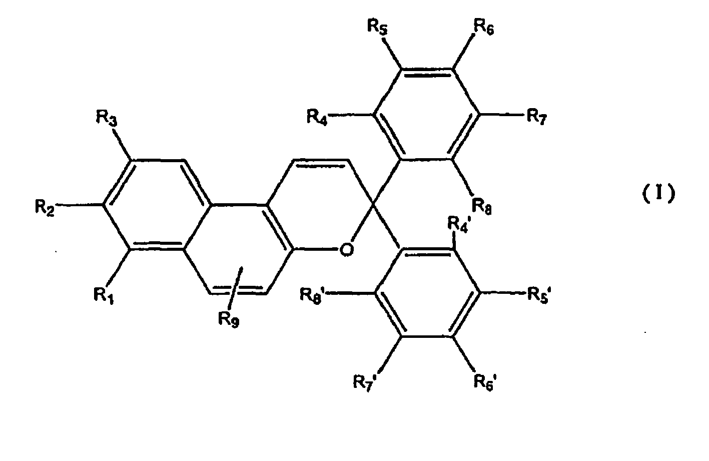 dans laquelle :
- R₁, R₂ et R₃ sont tels que :
   • soit, l'un ou deux d'entre eux comprend un groupe divalent (co)polymérisable avec un monomère, choisi dans le groupe constitué de :
      - un groupe comprenant une fonction vinylique choisi parmi
         -(CₙH₂ₙ)-O-(CH₂)_{n'}-CH=CH₂ et -(CₙH₂ₙ)-O-CH=CH₂
      - un groupe époxyde
      - un groupe (méth)acryloxy choisi parmi
         -(CₙH₂ₙ)-OC(=O)CH=CH₂ et -(CₙH₂ₙ)-OC(=O)C(CH₃)=CH₂,
      - un groupe amino primaire
      - un anhydride
      et le ou les autres groupes R₁, R₂ et R₃ sont choisis parmi H, (CₙH₂ₙ)-CH₃, (CₙH₂ₙ)-OH, n et n' étant compris entre 0 et 15,
   • soit au moins deux d'entre eux comprennent au moins un groupe monovalent(co)polymérisable avec un monomère, le troisième groupe étant, le cas échéant, choisi dans le groupe constitué de l'hydrogène, des halogènes et des alkyles en C1 à C15,
- R₄ à R₈ et R₄' à R₈' sont identiques ou différents et représentent indépendamment un hydrogène, un halogène, un hydroxyle, un alkyle en C1 à C15, un hydroxyalkyle en C1 à C15, un alcoxy en C1 à C15.
- R₉ représente un hydrogène, un hydroxyle, un alkyle en C1 à C15, un hydroxyalkyle en C1 à C15.

Les composés de formule (I), ont pour particularité de présenter, sur le noyau benzénique du groupement naphténique le plus éloigné du cycle pyrane, des substituants susceptibles de permettre à ces composés de participer à des réactions de (co)polymérisations en chaîne ou par étape avec un monomère. Ceci est rendu possible par le fait que les groupes R₁, R₂ et R₃ sont tels que l'un au moins est un groupe divalent (co)polymérisable avec un monomère ou que deux au moins de ces groupes comprennent un groupe monovalent (co)polymérisable avec un monomère.

Dans le cas où le groupe polymérisable est divalent, l'incorporation puis l'ancrage du monomère photochromique dans la matière se fera par polymérisation en chaîne (anionique, cationique, radicalaire, ouverture de cycle, métathèse). Les autres groupes R₁, R₂ et R₃ inertes vis-à-vis du mécanisme de polymérisation en chaîne peuvent être choisis parmi les alkyles, l'hydrogène, les halogènes, les hydroxyles, les alcoxy, les amines, les acides carboxyliques, les isocyanates, les silanes, qui ne peuvent pas réagir avec le groupe polymérisable divalent. Ils peuvent être activés a posteriori pour une post-réticulation par polycondensation ou polyaddition ou bien pour une nouvelle réaction de greffage.

Dans le cas où le groupe polymérisable est monovalent, la synthèse du polymère photochromique se fait par polymérisation par étapes entre monomères ou oligomères difonctionnels, c'est-à-dire portant au moins deux substituants monovalents. On peut former alors, par polycondensation ou polyaddition, notamment des polyuréthanes, des polyesters, des polyéthers, des polyamides ou des polysiloxanes. Le groupe inerte restant doit être non réactif vis-à-vis du mécanisme de polymérisation par étapes et est choisi parmi l'hydrogène, les alkyles ou les halogènes.

Dans le cas spécifique des groupes divalents anhydride cyclique et amine primaire, la polymérisation suit un mécanisme par étape ; l'ancrage du monomère photochromique se fait par polycondensation ou polyaddition d'un monomère photochromique de formule (I) dont au moins un des substituants R₁, R₂ et R₃ porte au moins un groupe anhydride ou amine primaire avec un monomère ou oliaomère difonctionnel.

Dans la formule (I), les groupements R₄ à R₈ et R₄' à R₈' sont avantageusement choisis indépendamment dans le groupe constitué de l'hydrogène, du méthyle, du méthoxy et du fluor et R₉ est avantageusement l'hydrogène.

En fonction de la nature des fonctions polymérisables portées par le cycle naphténique, les monomères photochromiques de formule (I) peuvent être impliqués dans des réactions de polymérisation en chaîne ou de polymérisation par étape.

Plus précisément, le monomère de formule (I) peut être impliqué dans une réaction de polymérisation en chaîne soit lorsqu'il est sous la forme d'un monomère divalent copolymérisable, soit lorsqu'il est sous la forme d'un monomère tétravalent copolymérisable qui peut alors servir d'agent de réticulation.

Un exemple de cas où le monomère de formule (I) se comporte comme un monomère divalent copolymérisable est celui où l'un des groupes R₁, R₂ et R₃ comprend une fonction vinylique, un groupe époxyde, un groupe (méth)acryloxy-, un groupe amino- primaire, un anhydride et les deux autres sont choisis parmi un hydrogène, un halogène, un hydroxyle, un groupe alkyle en C1 à C15, un groupe hydroxyalkyle en C1 à C15, un alcoxy en C1 à C15.

Un exemple de cas où le monomère de formule (I) se comporte comme un monomère tétravalent copolymérisable est celui où deux des groupes R₁, R₂ et R₃ comprennent une fonction vinylique, un époxyde, un groupe méth(acryloxy), un amino- primaire, un anhydride et le troisième groupe est un hydrogène, un halogène, un hydroxyle, un groupe alkyle en C1 à C15, un hydroxyalkyle en C1 à C15.

Dans les deux cas ci-dessus, l'un ou deux des groupes R₁, R₂ et R₃ sont avantageusement choisis dans le groupe constitué de -(CₙH₂ₙ)-OC(=O)CH=CH₂, -(CₙH₂ₙ)-OC(=O)C(CH₃)=CH₂.

-(CₙH₂ₙ)-O-(CH₂)_{n'}-CH=CH_{2'}, -(CₙH₂ₙ)-O-CH=CH₂, et les autres groupes R₁, R₂ et R₃ sont H, (CₙH₂ₙ)-CH₃, (CₙH₂ₙ)-OH, n et n' étant compris entre 0 et 15.

Par ailleurs, les monomères de formule (I) peuvent être impliqués dans des réactions de polymérisations par étape, en particulier lorsqu'ils sont sous la forme de monomères divalents (co)polymérisables, c'est-à-dire qu'ils portent deux groupes monovalents, ou de monomères trivalents copolymérisables pouvant servir d'agent de réticulation.

Un exemple de tels monomères divalents (co)polymérisables est celui des monomères de formule (I) dans lesquels deux des groupes R₁, R₂ et R₃ comprennent un hydroxyle, un groupe porteur d'un acide carboxylique, un hydroxyalkyle en C1 à C15, un isocyanato-, un époxyde, un amino-, un anhydride, ou un groupement silane réactif, ces deux groupes pouvant être identiques ou différents et le troisième groupe est choisi parmi un hydrogène ou un alkyle en C1 à C15, un halogène.

Un exemple de monomère de formule (I) trivalent copolymérisable est celui dans lequel les trois groupes R₁, R₂ et R₃ sont choisis indépendamment dans le groupe constitué des hydroxyles, des hydroxyalkyles, des isocyanato-, d'un anhydride, des époxydes, des amino-, des groupes porteurs d'un acide carboxylique, et des groupes porteurs d'un silane réactif (Si-H ou Si-C=C).

A titre de monomère de formule (I) préféré comprenant au moins deux groupements polymérisables monovalents, on peut citer ceux dans lesquels au moins deux des groupes R₁, R₂ et R₃ sont choisis parmi les groupes -(CₙH₂ₙ)-OH, -(CₙH₂ₙ)-NH₂, -(CₙH₂ₙ)-[CH-CH₂-O]cycle, -(CₙH₂ₙ)-COOH, -(CₙH₂ₙ)-Si(CₘH₂ₘ)₂-H et -(CₙH₂ₙ)-Si-CHCH₂, -(CₙH₂ₙ)-Si-(O-CₘH₂ₘ)₃, -(CₙH₂ₙ)-OC(=O)NH- R10-N=C=O, avec R₁₀ étant choisi parmi -(CₙH₂ₙ), -(CₙH₂ₙ₋₂),

-(CₙH₂ₙ₂)-CH₂-(CₙH₂ₙ₋₂), aryle ou aryle-CH₂-aryle, n étant compris entre 0 et 15, m étant compris entre 1 et 15 et les groupes aryle comprenant de 5 à 20 atomes de carbone ; le groupe restant, s'il y en a, étant H ou un alkyle en C1 à C15.

Parmi ces composés, ceux comprenant trois groupements R₁, R₂ et R₃ choisis indépendamment dans les groupes tels que définis ci-dessus, permettent d'accéder à des monomères trivalents copolymérisables pouvant servir d'agent de réticulation.

En ce qui concerne les monomères de formule (I) comprenant deux groupes R₁, R₂ et R₃ polymérisables monovalents, il est à noter que les fonctions polymérisables peuvent être soit de même nature, soit de nature différente, par exemple :
- un hydroxyalkyle et un groupe amine, le troisième groupe étant l'hydrogène ou un groupe alkyle.
- un groupe hydroxyalkyle et un groupe porteur d'un acide carboxylique, le troisième groupe étant soit de l'hydrogène, soit un groupe alkyle,
- un groupe porteur d'un acide carboxylique et un groupe amine, le troisième groupe étant lui-même soit un hydrogène, soit un alkyle,

Selon un aspect tout particulièrement préféré de l'invention, ledit monomère photochromique répond à la formule : 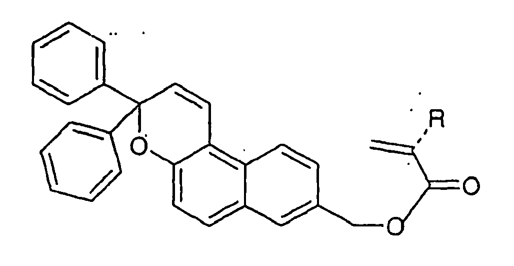 dans laquelle R représente H ou CH₃.

Les composés de formule (I) décrits ci-dessus peuvent être préparés par une voie de synthèse qui comprend une étape de cyclisation que l'on désignera ci-après par étape de chroménisation, au cours de laquelle on réalise la précipitation d'un produit intermédiaire répondant à la formule Il ci-dessous : 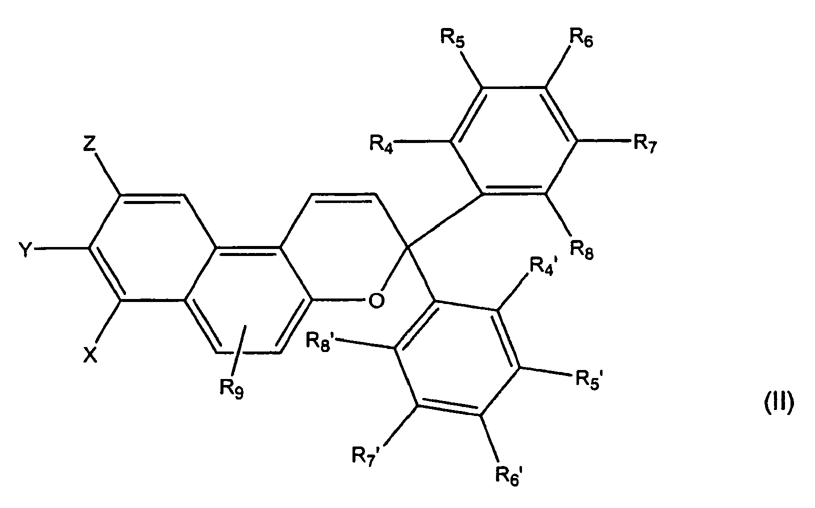 dans laquelle les groupements X, Y, Z sont soit les groupes R₁, R₂ et R₃ tels que définis précédemment, soit des précurseurs éventuellement protégés de ces groupements, les différents groupes R₄ à R₉ et R₄' à R₈' étant tels que définis précédemment.

On donne ci-dessous le schéma de synthèse complet (schéma de synthèse n°1) des monomères préférés de l'invention, dans lequel le groupe R₂ est un groupe polymérisable divalent de type (méth)acrylique :

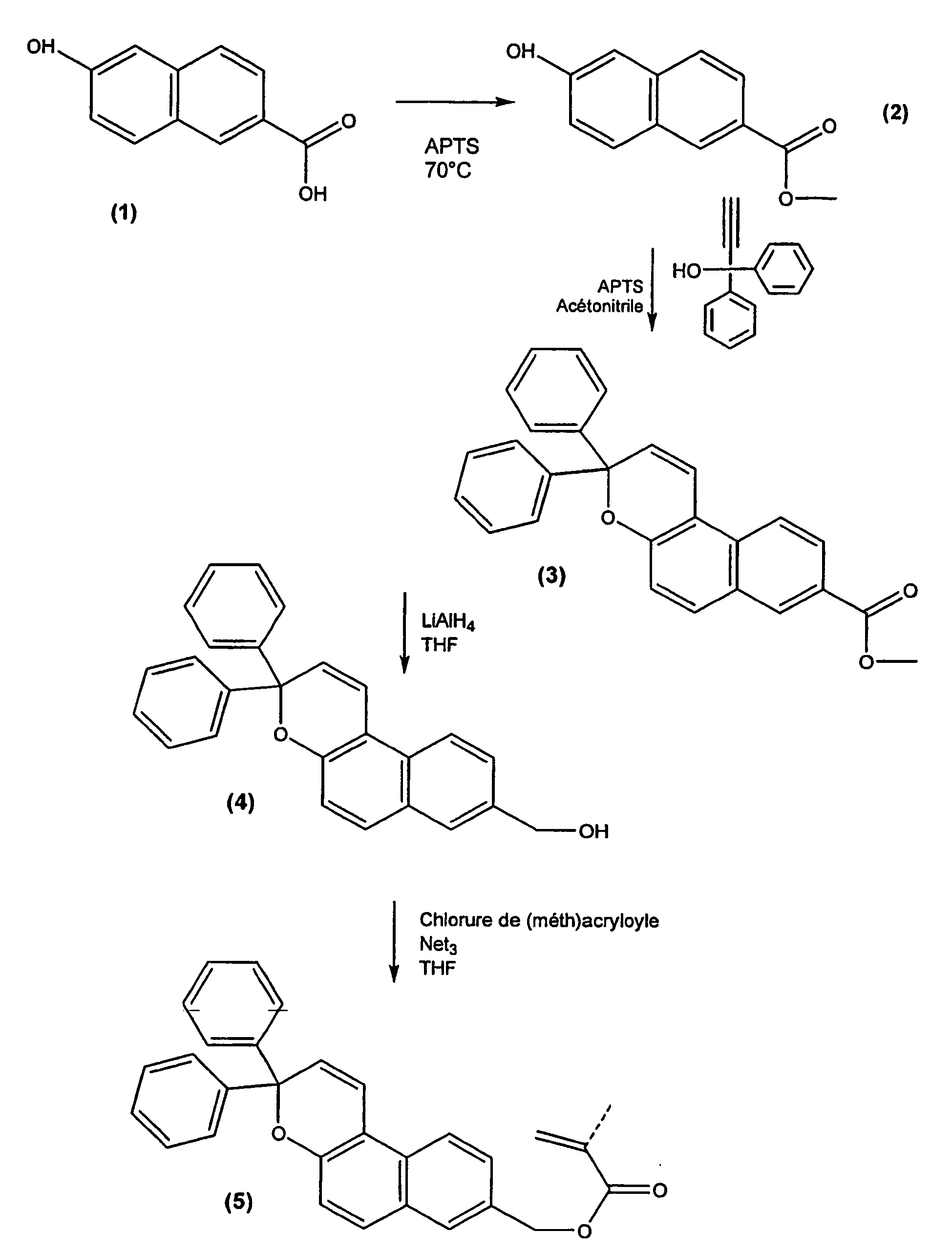

Comme cela apparaît sur le schéma de synthèse ci-dessus, qui fait l'objet d'une description détaillée dans les exemples 1 et 2 qui suivent, le produit final (produit 5) est obtenu dans des étapes parfaitement industrialisables du fait du bon rendement de chacune de ces étapes et de la pureté des produits obtenus.

Le produit (3) peut être isolé de façon particulièrement simple et efficace par une simple filtration du fait du choix du milieu réactionnel dans lequel est réalisé la cyclisation (chroménisation) permettant de le synthétiser à partir du composé (2) de l'étape précédente.

Cette étape de cyclisation au cours de laquelle on précipite l'intermédiaire recherché pour les étapes ultérieures s'avère être une étape clé du procédé.

En effet, c'est à partir de ce produit (3) que l'on pourra fabriquer bon nombre des monomères de formule (I) décrits plus haut.

En effet, dans le cas du schéma ci-dessus, le composé (3) est soumis à une étape de réduction pour conduire au produit (4) qui subit ensuite un greffage.

L'homme du métier comprendra aisément au vu de ce schéma que l'on peut envisager d'autres étapes de « déprotection », par exemple l'hydrolyse de la fonction ester pour obtenir un acide carboxylique ou encore la réduction contrôlée de l'ester en aldéhyde.

On peut également envisager d'autres étapes de greffage, par exemple la réaction du produit hydroxylé (4) avec un diisocyanate pour obtenir un photochrome à fonction réactive isocyanate ou encore la fonctionnalisation du produit (4) par réaction de transéthérification avec un éther énolique en présence d'acétate de mercure pour conduire à une fonction vinylique.

Par ailleurs, l'homme du métier comprendra aisément que l'on peut obtenir dans un schéma analogue à celui représenté ci-dessus un groupe époxy- par réaction du composé (4) avec de l'épichlorhydrine.

Ainsi, par un schéma analogue à celui présenté ci-dessus, on peut accéder à l'ensemble des monomères de formule (I) présentant un groupement polymérisable divalent et cela, à partir d'un produit commercial tel que le produit (1) ou de produits dérivés de ce produit pour lesquels par exemple le groupement R₁ ou R₃ porte un groupement CO₂H.

En ce qui concerne les monomères de l'invention comprenant deux groupements monovalents, ils peuvent être obtenus en suivant le schéma de synthèse ci-dessous (schéma de synthèse n° 2) ou un schéma dérivé de celui-ci, aisément envisageable par l'homme du métier.

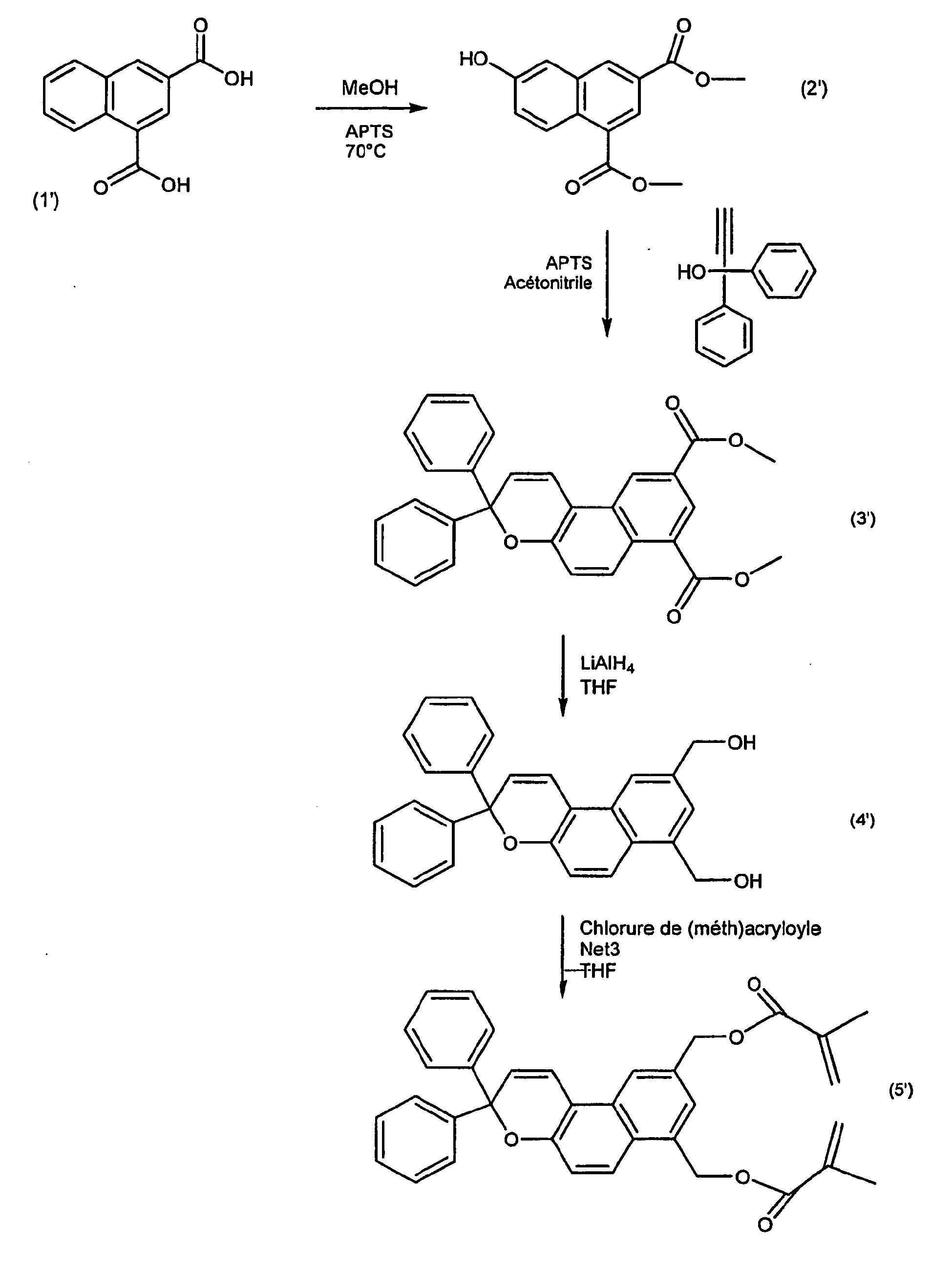

Comme dans le cas précédent, d'autres types de fonctions peuvent être envisagées, ainsi le groupe hydroxyle du composé (4') peut être transformé en amine ou l'ester (3') hydrolysé en acide carboxylique.

Outre le (ou les) monomères photochromique(s), le polymère selon l'invention peut être formé d'au moins un autre monomère, non photochromique, de formule (III)

CH2=C(R₁₁)-COO-(CH₂)ₚ-Y (III)

dans laquelle :
* R₁₁ est H ou CH₃
* p est 0-10
* Y est choisi dans le groupe constitué par : l'hydrogène ; OH ; un groupe hydroxyalkyle en C1-C10 ; un groupe fluoroalkyle en C1-C10 ; un groupe alkoxy en C1-C10 ; un groupe acyloxy en C1-C10 ; un groupe acylamino en C1-C10 ; un groupe alkylsulfinyle en C1-C10 ; un groupe alkylsulfonyle en C1-C10 ; un reste -(CH2-CH2-O)ₜ-H où t est compris entre 1 à 100 ; un reste Ar ou W-Ar, Ar représentant un noyau aromatique, notamment phényle, le cas échéant substitué par un groupe alkyle en C1-C8, un atome de chlore ou un atome de brome et W représentant O, S ou NR₁₂, R₁₂ étant H ou un groupe alkyle en C1-C10.

Le monomère de formule (III) est de préférence choisi dans le groupe constitué par un méthacrylate d'alkyle, de préférence le méthacrylate de méthyle, un hydroxyalkyl méthacrylate, un hydroxyalkyl acrylate, un phénoxyalkyl acrylate et un phénoxyalkyl méthacrylate.

Plus préférentiellement, le monomère de formule (III) est l'hydroxyethyl méthacrylate ou le 2-phénoxyéthyl méthacrylate.

Selon un aspect avantgeux de l'invention, le polymère formant tout ou partie de la lentille intaocculaire comprend :
* un monomère photochromique consistant en le méthacrylate de 3,3 diphényl-3H naphto [2,1]-pyrane, l'acrylate de 3,3 diphényl-3H naphto [2,1]- pyrane ou un mélange de ces monomères, et
* l'hydroxyethyl méthacrylate ou le 2-phénoxyéthyl méthacrylate. Les polymères conformes à l'invention peuvent être obtenus par copolymérisation d'un monomère photochromique, en particulier un monomère de formule (I) décrit ci-dessus, avec au moins un monomère non photochromique, en particulier un monomère de formule (III) décrit ci-dessus.

La polymérisation peut se faire selon toute méthode conventionnelle, en particulier celles indiquées plus haut, par exemple polymérisation en chaîne (anionique, cationique, radicalaire, ouverture de cycle, métathèse) ou polymérisation par étape.

La polymérisation peut être réalisée en présence d'agent réticulant, tel que les diacrylates et diméthacrylates de bisphénol A éthoxylate(1 EO/phénol), bisphénol A éthoxylate (2 EO/phénol), bisphénol A propoxylate (2 PO/phénol), bisphénol A, 2,2'-diallylbisphénol A ou bis(4-(2-methacryloyléthoxy)phényl)méthane.

La polymérisation peut être également réalisée en présence de composés d'initiation de polymérisation qui peuvent être ajoutés aux monomères, en particulier, des peroxydes, des peroxydiacarbonates, des radicaux azo libres, notamment le 2,2-azobisisobutyronitrile.

Habituellement, un polymère constitutif d'une lentille intraoculaire selon l'invention, comporte une proportion de monomères photochromiques dans ledit polymère comprise entre 0,01 % et 5 %, de préférence entre 0,02 % et 2 % (proportion massique).

La polymérisation peut être réalisée directement dans une moule de sorte qu'on récupère le polymère sous forme d'uné ébauche de lentille intraoculaire.

Cette ébauche peut être ensuite mise sous forme d'une lentille intraoculaire selon des méthodes conventionnelles pour l'homme du métier, par exemple par usinage ou cryo usinage sur tour. Les polymères utilisés dans le cadre de l'invention sont, quand ils sont excités par la lumière, de préférence de couleur jaune à orange.

Lorsque la lumière diminue, le polymère perd sa couleur. Dans ces conditions, la lentille intraoculaire de l'invention, comportant en tout ou partie un tel polymère, permet de protéger la rétine contre les rayons lumineux, notamment les bleus et les rayons UV, tout en autorisant une vision de bonne qualité quand l'intensité lumineuse diminue.

Les exemples qui suivent ont pour but d'illustrer l'invention.

### EXEMPLES

### Exemple 1 : Synthèse du 8-hydroxymethyl-3,3, diphényl-3H naphto [2,1]-pyrane (composé (4))

Cet exemple est donné en référence au schéma de synthèse n°1, décrit plus haut.

### Etape 1 : Estérification du composé (1)

Dans un ballon à trois tubulures (250 ml), surmonté d'un condenseur, le composé (1) (7g) est dissout dans 80 ml de méthanol. La réaction d'estérification est catalysée par l'ajout d'acide paratoluènesulfonique (APTS, 0.48g) introduit sous azote. La réaction est menée à une température de 70°C et est laissée sous agitation au moins 12 heures.

A l'issue de ce délai, le méthanol est évaporé et la phase organique résiduelle est dissoute dans de l'acétate d'éthyle en vue d'extractions liquide/liquide (acétate d'éthyle/ eau saturée en carbonate de potassium) afin de purifier l'ester (composé (2)) attendu.

La phase organique résultante des différents lavages est ensuite séchée sur MgSO4 puis filtrée. L'évaporation de l'acétate d'éthyle permet d'isoler de manière quantitative l'ester recherché (6 à 7 g).

### Etape 2 : Chroménisation du composé (2)

Dans un ballon à trois tubulures (100 ml), surmonté d'un condenseur, on introduit 1.56g de composé (2) avec 50 ml d'acétonitrile.

Le milieu devient limpide dès la température de 50°C atteinte, moment où l'on ajoute, sous azote, 1.6g (1eq/(2)) d'alcool propargylique et 0.122g d'APTS (0.08eq/(2)). Le milieu réactionnel est refroidi à température ambiante et on agite pendant deux jours à cette température.

Le composé (3) est simplement isolé par filtration puisque insoluble dans le milieu réactionnel. Il est purifié par lavages dans l'acétonitrile à 40°C suivis de filtrations. Le produit est obtenu avec un rendement de 55% sans purification supplémentaire.

### Etape 3 : Réduction de l'ester (3) pour atteindre (4) : 8-hydroxymethyl-3,3 diphényl-3H naphto[2,1]-pyrane

Dans un ballon à trois tubulures (100 ml), muni d'un bulleur, on introduit 0.2g de LiAlH₄ (1.41eq/(3)) dilué dans 15 ml de THF anhydre. On ajoute ensuite, sous azote et goutte à goutte les 1.5 g de (3) dissous dans 30ml de THF anhydre. Lors de l'addition aucun dégagement gazeux 20 violent n'est observé. Le tout est agité au moins 12 heures à température ambiante.

Avant d'isoler le produit (4), l'excès de LiAlH₄ doit être neutralisé. Pour ce faire, on ajoute lentement 1.25 ml d'eau puis 12.5ml d'une solution d'acide sulfurique (H₂SO₄) à 10%. L'ajout d'éther dans le milieu fait apparaître deux phases. La phase organique extraite est lavée (eau saturée en NaCl) puis séchée avec MgSO4, filtrée et le solvant évaporé.

Le produit blanc obtenu avec un rendement quantitatif, correspond au composé (4) attendu.

### Exemple 2 : Fonctionnalisation avec le chlorure d'acryloyle : composé (5)

Dans un ballon à trois tubulures (100 ml), surmonté d'un condenseur, on introduit 1g de composé 4, 0.5mL de Et₃N (1.3eq/ (4)) dissout dans 30mL de THF anhydre. L'addition du chlorure est faite goutte à goutte, sous azote, à température ambiante, sans observer d'élévation de température importante. La réaction est laissée à température ambiante et sous agitation pendant au moins 12 heures.

A l'issue de cette période, le solvant est évaporé. Le résidu est dilué dans le dichlorométhane puis extrait par lavages successifs avec de l'eau saturée en carbonate de potassium. La phase organique extraite est séchée avec MgSO4, filtrée sur silice et le solvant évaporé.

L'acrylate de 3,3 diphenyl-3H naphto[2,1]-pyrane est isolée avec un rendement massique de 60%.

### Exemple 3 : Fonctionnalisation avec le chlorure de méthacryloyle

La synthèse est identique à celle de l'exemple 2, sauf que le chlorure d'acryloyle est remplacé par le chlorure de méthacryloyle

On obtient ainsi le méthacrylate de 3,3 diphenyl-3Hnaphto[2,1]-pyrane.

### Exemple 4 : élaboration d'un polymère photochromique servant de matière première aux lentilles intraoculaires à partir du monomère de l'exemple 3.

Dans un tube de verre, on introduit 72,439g d'hydroxyethylméthacrylate distillé suivie de 10,08g de méthacrylate de méthyle distillé, ainsi que 0,210g d'ethyleneglycoldiméthacrylate. L'amorceur choisi est le 2,2-azobisiobutyronitrile qui est solubilisé dans le mélange de monomère (0,167g). 0,041g de méthacrylate de 3,3 diphenyl-3H naphto[2,1]-pyrane (produit de l'exemple 3) est ajouté au milieu réactionnel. Ce dernier est placé sous atmosphère inerte et est soumis au cycle de température suivant : 24h à 48°C, 9h à 80°C et 3h de post cuisson à 90°C.

### Exemple 5: Elaboration d'un polymère photochromique servant de matière première aux lentilles intraoculaires à partir du monomère de l'example 2.

Dans un tube de verre, on Introduit 72,439g d'hydroxyethylméthacrylate distillé suivie de 10,06g de méthacrylate de méthyle distillé ainsi que 0,210g d'éthyleneglycoldiméthacrylate. L'amorce choisi est le 2,2-azobisiobutyronitrile qui est solubilisé dans le mélange de monomère (0,167g) avec 0,04g d'acrylate de 3,3 diphenyl-3H, naphto[2,1]-pyrane (produit de l'exemple 2).

Ce dernier est placé sous atmosphère inerte et est soumis au cycle de température suivant : 24h à 48°C, 9h à 80°C et 3h de post cuisson à 90°C.

Le polymère obtenu présente, après hydratation une teneur en eau de 28%. Incolore, il développe réversiblement une couleur jaune lorsqu'on le soumet à la lumière du soleil.

Le polymère a été soumis à des tests d'absorbance en forme fermée et en forme ouverte. Les résultats obtenus sont représentés à la figure 1.

### Exemple 6: Elaboration d'un polymère photochromique servant de matière première aux lentilles intraoculaires à partir du monomère de l'exemple 3.

Dans un tube en verre, on introduit 82g de méthacrylate de méthyle et 0,21g d'éthylène glycoldimethacrylate. L'amorceur choisi est le 2,2-azobisisobutyronitrile solubilisé dans le mélange de monomère (0,167g) avec 0,04g de méthacrylate de 3,3 diphényl-3H, naphto [2,1]-pyrane (produit de l'exemple 3).

### Exemple 7: Elaboration d'un polymère photochromique servant de matière première aux lentilles intraoculaires à partir du monomère de l'exemple 2.

Dans un ballon à 3 tubulures (2L), on introduit 550,0 g de 2-phénoxyéthyl méthacrylate, 0,286 g d'acrylate de 3,3-diphényl-3H naphto [2,1]-pyrane (produit de l'exemple 2) et 716,15±10,0 mg de 2,2'-azobisisobutyronitrile. Une fois les particules solides dissoutes dans le monomère liquide, le mélange est agité à 800tr/min sous azote pendant 45 minutes à température ambiante. La température du mélange est alors portée à 45±2°C et la vitesse d'agitation progressivement diminuée à 150tr/min au fur et à mesure que la viscosité du mélange augmente. Lorsque la disparition du vortex est observée, la polymérisation est stoppée par un refroidissement rapide du milieu réactionnel avec de l'azote liquide. Lorsque le mélange est revenu à température ambiante on y ajoute 27.8g de bisphenol ethoxylate (2EO/phenol) diméthacrylate. Le milieu réactionnel est maintenu sous agitation pendant 2h à température ambiante. Le mélange est ensuite dégazé sous vide pendant 5h avant d'être filtré.

Le mélange est ensuite porté à une température de 118°C pendant 13 heures puis de 135° pendant 3 heures.

## Revendications

1. Une lentille intraoculaire **caractérisée en ce qu'**elle comporte au moins un polymère photochromique pharmaceutiquement acceptable permettant à ladite lentille intraoculaire, en tout ou partie, de changer de couleur réversiblement lorsqu'elle est exposée à la lumière, ledit polymère photochromique étant formé d'au moins un monomère photochromique qui est un composé du type 3,3-diaryl-3H-naphto[2,1-b]pyrane substitué polymérisable de formule (I) : 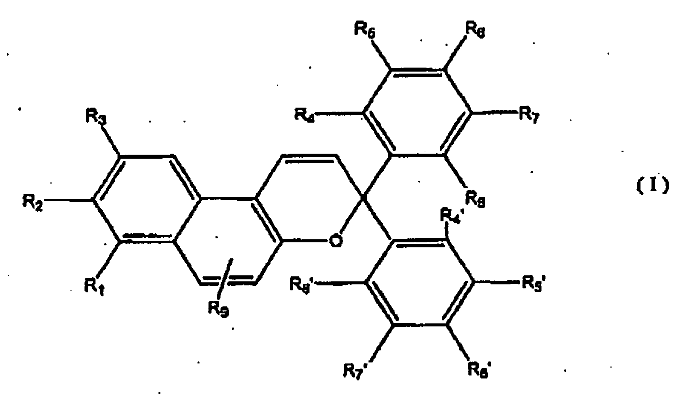 dans laquelle
- R₁-R₂ et R₃ sont tels que :
• soit, l'un ou deux d'entre eux comprend un groupe divalent (co)polymérisable avec un monomère, choisi dans le groupe constitué de :
• un groupe comprenant une fonction vinylique choisi parmi
-(CₙH₂ₙ)-O-(CH₂)ₙ-CH=CH₂ et -(CₙH₂ₙ)-O-CH=CH₂
• un groupe époxyde
• un groupe (méth)acryloxy choisi parmi
-(cₙH₂ₙ)-OC(=O)CH=CH₂ et -(CₙH₂ₙ)-OC(=O)C(CH₃)=CH₂,
• un groupe amino primaire
• un anhydride
et le ou les autres groupes R₁, R₂ et R₃ sont choisis parmi H, (CₙH₂ₙ)-CH₃, (CₙH₂ₙ)-OH, n et n' étant compris entre 0 et 15
• soit au moins deux d'entre eux comprennent au moins un groupe monovalent(co)polymérisable avec un monomère, le troisième groupe étant, le cas échéant, choisi dans le groupe constitué de l'hydrogène, des halogènes et des alkyles en C1 à C15,
- R₄ à Rₐ et R₄' à Rₐ' sont identiques ou différents et représentent indépendamment un hydrogène, un halogène, un hydroxyle, un alkyle en C1 à C15, un hydroxyalkyle en C1 à C15, un alcoxy en C1 à C15.
- R₉ représente un hydrogène, un hydroxyle, un alkyle en C1 à C15, un hydroxyalkyle en C1 à C15.

2. Lentille intraoculaire selon la revendication 1, **caractérisée en ce qu'**elle comporte une partie optique et une partie haptique constituée d'un ou plusieurs éléments, la partie optique au moins comportant ledit polymère photochromique pharmaceutiquement acceptable.

3. Lentille intraoculaire selon l'une des revendications 1 ou 2, **caractérisée en ce que**, dans la formule (I), R₄ à R₈ et R₄' à R₈' sont identiques ou différents et représentent indépendamment un hydrogène, un méthyle, un méthoxy ou du fluor et R₉ est l'hydrogène.

4. Lentille intraoculaire selon l'une quelconque des revendications 1 à 3, **caractérisée en ce que**, dans la formule (I), deux des groupes R₁, R₂ et R₃ comprennent un hydroxyle, un groupe porteur d'un acide carboxylique, un hydroxyalkyle en C1 à C15, un isocyanato-, un époxyde, un amino-, un anhydride, ou un groupement silane réactif, ces deux groupes pouvant être identiques ou différents et le troisième groupe est choisi parmi un hydrogène ou un alkyle en C1 à C15, un halogène.

5. Lentille intraoculaire selon l'une quelconque des revendications 1 à 3, **caractérisée en ce que**, dans la formule (I), les trois groupes R₁, R₂ et R₃ sont choisis indépendamment dans le groupe constitué des hydroxyles, des hydroxyalkyles, des isocyanato-, d'un anhydride, des époxydes, des amino-, des groupes porteurs d'un acide carboxylique, et des groupes porteurs d'un silane réactif.

6. Lentille intraoculaire selon l'une des revendications 4 ou 5, **caractérisée en ce qu'**au moins deux des groupes R₁, R₂ et R₃ sont choisis parmi les groupes-(CₙH₂ₙ)-OH, -(CₙH₂ₙ)-NH₂, -(CₙH₂ₙ)-[CH-CH₂-O]cycle, -(CₙH₂ₙ)-COOH, -(CₙH₂ₙ)-Si(CₘH₂ₘ)₂-H et -(CₙH₂ₙ)-Si-CHCH₂, -(CₙH₂ₙ)-Si-(O-CₘH₂ₘ)₃, -(CₙH₂ₙ)-O-C(=O)NH- R₁₀-N=C=O, avec :
R₁₀ = -(CₙH₂ₙ), ou -(CₙH₂ₙ₋₂) ou -(CₙH₂ₙ₂)-CH₂-(CₙH₂ₙ₋₂), aryle ou aryle-CH₂-aryle,
n étant compris entre 0 et 15,
m étant compris entre 1 et 15 et
les groupes aryle comprenant de 5 à 20 atomes de carbone ;
le groupe restant étant, le cas échéant, H ou un alkyle en C1 à C15.

7. Lentille intraoculaire selon la revendication 6, **caractérisée en ce que** les groupes R₁, R₂ et R₃ sont choisis indépendamment parmi les groupes -(CₙH₂ₙ)-OH, -(CₙH₂ₘ)-NH₂, -(CₙH₂ₙ)-[CH-CH₂-O]cycle,
-(CₙH₂ₙ)-COOH, -(CₙH₂ₙ)-Si(CₘH₂ₘ)₂-H et-(CₙH₂ₙ)-Si-CH=CH₂,
-(CₙH₂ₙ)-Si-(O-CₘH₂ₘ)₃, -(CₙH₂ₙ)-O-C(=O)NH- R₁₀-N=C=O, avec :
R₁₀ = -(CₙH₂ₙ), ou -(CₙH₂ₙ₋₂) OU -(CₙH₂ₙ₋₂)-CH₂-(CₙH₂ₙ₋₂), aryle ou aryle-CH₂-aryle,
n étant compris entre 0 et 15
m étant compris entre 1 et 15 et
les groupes aryle comprenant de 5 à 20 atomes de carbone.

8. Lentille intraoculaire selon l'une des revendications 1 ou 2, **caractérisée en ce que** ledit monomère photochromique répond à la formule : 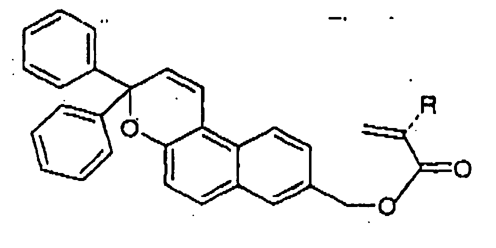 dans laquelle R représente H ou CH₃.

9. lentille intraoculaire selon l'une quelconque des revendications 1 à 8, **caractérisée en ce que** ledit polymère est formé, en outre, d'au moins un monomère non photochromique de formule (III)
CH2=C(R₁₁)-COO-(CH₂)ₚ-Y (III)
dans laquelle :
* R₁₁ est H ou CH₃
* p est 0-10
* Y est choisi dans le groupe constitué par : l'hydrogène ; OH ; un groupe hydroxyalkyle en C1-C10: un groupe fluoroalkyle en C1-C10 ; un groupe alkoxy en C1-C10 ; un groupe acyloxy en C1-C10 ; un groupe acylamino en C1-C10 ; un groupe alkylsulfinyle en C1-C10 ; un groupe alkylsulfonyle en C1-C10 ; un reste -(CH2-CH2-O)ₜ-H où t est compris entre 1 à 100 ; un reste Ar ou W-Ar, Ar représentant un noyau aromatique, notamment phényle, le cas échéant substitué par un groupe alkyle en C1-C8, un atome de chlore ou un atome de brome et W représentant O, S ou NR₁₂, R₁₂ étant H ou un groupe alkyle en C1-C10.

10. Lentille intraoculaire selon la revendication 9, **caractérisée en ce que** le monomère de formule (III) est choisi dans le groupe constitué par :
un méthacrylate d'alkyle, de préférence le méthacrylate de méthyle, un hydroxyalkyl méthacrylate, un hydroxyalkyl acrylate, un phénoxyalkyl acrylate et un phénoxyalkyl méthacrylate.

11. Lentille intraoculaire selon la revendication 10, **caractérisée en ce que** le monomère de formule (III) est l'hydroxyethyl méthacrylate ou le 2-phénoxyéthyl méthacrylate.

12. Lentille intraoculaire selon l'une des revendications 1 à 3, **caractérisée en ce que** ledit polymère comprend :
* un monomère photochromique consistant en le méthacrylate de 3,3 diphényl-3H naphto [2,1]-pyrane, l'acrylate de 3,3 diphényl-3H naphto [2,1]- pyrane ou un mélange de ces monomères, et
* l'hydroxyethyl méthacrylate ou le 2-phénoxyéthyl méthacrylate.

13. Lentille intraoculaire selon l'une quelconque des revendications. 1 à 12, **caractérisée en ce que** la proportion du monomère photochromique dans le polymère est comprise entre 0,01% et 5%, de préférence entre 0,02 et 2% (proportion massique).

## Claims

1. Intraocular lens, **characterized in that** it comprises at least one pharmaceutically acceptable photochromic polymer that allows all or part of said intraocular lens to change color reversibly when exposed to light, wherein said phtochromic polymer is formed of at least one photochromic monomer which is a compound of the polymerizable substituted 3,3-diaryl-3H-naphtho [2,1-b]pyran type, of formula (I): 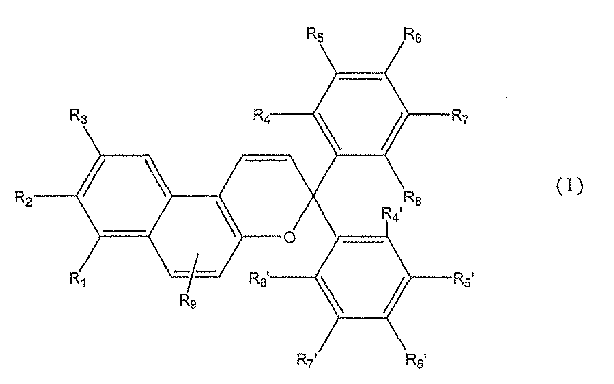 in which
- R₁, R₂ and R₃ are such that:
* either at least one of them comprises a divalent group which is (co)polymerizable with a monomer, selected from the group consisting of
■ a group comprising a vinyl function selected from
- (CₙH₂ₙ)-O-(CH₂) _{n'} -CH=CH₂ and
- (CₙH₂ₙ )-O-CH=CH₂
■ an epoxide
■ a (meth) acryloxy group selected from
- (CₙH₂ₙ) -O-C (=O) CH=CH₂ and
- (CₙH₂ₙ) -O-C (=O) C (CH₃) =CH₂,
■ a primary amino group, and
■ an anhydride
and the other group (s) R₁, R₂ and R₃ are chosen from hydrogen, (CₙH₂ₙ)-CH₃, (CₙH₂ₙ) -OH, where n and n' are between 0 and 15,
* or at least two of them comprise at least one monovalent group which is (co)polymerizable with a monomer, the third group being, where appropriate, chosen from the group consisting of hydrogen, halogens and C₁ to C₁₅ alkyls,
- R₄ to R₈ and R'₄ to R'₈ are identical or different and independently represent a hydrogen, a halogen, a hydroxyl, a C₁ to C₁₅ alkyl, a C₁ to C₁₅ hydroxyalkyl or a C₁ to C₁₅ alkoxy,
- R₉ represents a hydrogen, a hydroxyl, a C₁ to C₁₅ alkyl or a C₁ to C₁₅ hydroxyalkyl.

2. Intraocular lens according to claim 1, **characterized in that** it comprises an optical part and a haptic part consisting of one or more elements, the optical part at least comprising said pharmaceutically acceptable photochromic polymer.

3. Intraocular lens according to claims 1 or 2, **characterized in that**, in formula (I), R₄ to R₈ and R'₄ to R'₈ are identical or different and independently represent a hydrogen, a methyl, a methoxy or fluorine and R₉ is hydrogen.

4. Intraocular lens according to either of claims 1 to 3, **characterized in that**, in formula (I), two of the groups R₁, R₂ and R₃ comprise a hydroxyl, a group bearing a carboxylic acid, a C₁ to C₁₅ hydroxyalkyl, an isocyanato-, an epoxide, an amino-, an anhydride, or a reactive silane group, it being possible for these two groups to be identical or different, and the third group is chosen from a hydrogen, a C₁ to C₁₅ alkyl and a halogen.

5. Intraocular lens according to either of claims 1 to 3, **characterized in that**, in formula (I), the three groups R₁, R₂ and R₃ are chosen independently from the group consisting of hydroxyls, hydroxylalkyls, isocyanato- groups, an anhydride, epoxides, aminogroups, groups bearing a carboxylic acid, and groups bearing a reactive silane.

6. Intraocular lens according to either of claims 4 or 5, **characterized in that** at least two of the groups R₁, R₂ and R₃ are chosen from the groups -(CₙH₂ₙ)-OH, -(CₙH₂ₙ)-NH₂, -(CₙH₂ₙ)-[CH-CH₂-O] ring, -(CₙH₂ₙ)-COOH, -(CₙH₂ₙ)-Si(CₘH₂ₘ) ₂-H and -(CₙH₂ₙ) Si-CHCH₂, -(CₙH₂ₙ)-Si-(O-CₘH₂ₘ)₃, -(CₙH₂ₙ)-O-C(=O) H-R₁₀-N=C=O, with:
R₁₀ =-(CₙH₂ₙ), -(CₙH₂ₙ-₂), -(CₙH₂ₙ₂)-CH₂-(CₙH₂ₙ₋₂), aryl or aryl-CH₂-aryl,
n being between 0 and 15,
m being between 1 and 15, and
the aryl groups comprising from 5 to 20 carbon atoms;
the remaining group being, where appropriate, H or a C₁ to C₁₅ alkyl.

7. Intraocular lens according to claim 6, **characterized in that** the groups R₁, R₂ and R₃ are chosen independently from the groups- (CₙH₂ₙ)-OH, -(CₙH₂ₙ)-NH₂, -(CₙH₂ₙ)-[CH-CH₂-O]ring, -(CₙH₂ₙ)-COOH, -(CₙH₂ₙ)-Si(CₘH₂ₘ)₂-H and -(CₙH₂ₙ)-Si-CH=CH₂, -(CₙH₂ₙ)-Si-(O-CₘH₂ₘ)₃, -(CₙH₂ₙ)-O-C(=O)NH-R₁₀-N=C=O, with:
R₁₀ = -(CₙH₂ₙ) or -(CₙH₂ₙ₋₂) or -(CₙH₂ₙ₋₂)-CH₂-(CₙH₂ₙ₋₂), aryl or aryl-CH₂-aryl,
n being between 0 and 15,
m being between 1 and 15 and
the aryl groups comprising from 5 to 20 carbon atoms.

8. Intraocular lens according to claim 1 or 2, **characterized in that** said photochromic monomer corresponds to the formula: 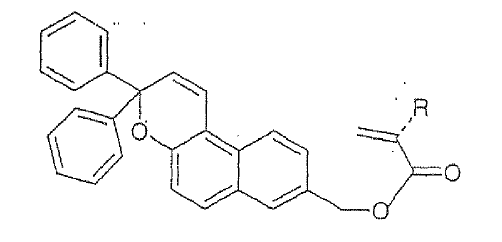 in which R represents H or CH₃.

9. Intraocular lens according to one of claims 1 to 8, **characterized in that** said polymer is also formed from at least one non-photochromic monomer of formula (III)
CH₂=C (R₁₁) -COO- (CH₂)ₚ-Y (III)
in which:
* R₁₁ is H or CH₃,
* p is 0-10,
* Y is chosen from the group consisting of: hydrogen; OH; a C₁-C₁₀ hydroxyalkyl group; a C₁-C₁₀ fluoroalkyl group; a C₁-C₁₀ alkoxy group; a C₁-C₁₀ acyloxy group; a C₁-C₁₀ acylamino group; a C₁-C₁₀ alkylsulfinyl group; a C₁-C₁₀ alkylsulfonyl group; a residue -(CH₂-CH₂-O)ₜ-H where t is between 1 and 100; a residue Ar or W-Ar, Ar representing an aromatic ring, in particular phenyl, where appropriate substituted with a C₁-C₈ alkyl group, a chlorine atom or a bromine atom, and W representing O, S or NR₁₂, R₁₂ being H or a C₁-C₁₀ alkyl group.

10. Intraocular lens according to claim 9, **characterized in that** the monomer of formula (III) is chosen from the group consisting of:
an alkyl methacrylate, preferably methyl methacrylate, a hydroxyalkyl methacrylate, a hydroxyalkyl acrylate, a phenoxyalkyl acrylate and a phenoxyalkyl methacrylate.

11. Intraocular lens according to claim 10, **characterized in that** the monomer of formula (III) is hydroxyethyl methacrylate or 2-phenoxyethyl methacrylate.

12. Intraocular lens according to one of claims 1 to 3, **characterized in that** said polymer comprises:
* a photochromic monomer consisting of 3,3-diphenyl-3H-naphtho[2,1]pyran methacrylate, 3,3-diphenyl-3H-naphtho[2,1]pyran acrylate or a mixture of these monomers, and
* hydroxyethyl methacrylate or 2-phenoxyethyl methacrylate.

13. Intraocular lens according to any one of claims 1 to 12, **characterized in that** the proportion of the photochromic monomer in the polymer is between 0.01% and 5%, preferably between 0.02% and 2% (proportion by mass).

## Patentansprüche

1. Intraokulare Linse, **dadurch gekennzeichnet, dass** sie wenigstens ein pharmazeutisch verträgliches photochromes Polymer umfasst, das der intraokularen Linse erlaubt, die Farbe reversibel, ganz oder teilweise, zu ändern, wenn sie dem Licht ausgesetzt wird, wobei das photochrome Polymer aus wenigstens einem photochromen Monomer gebildet wird, das eine substituierte polymerisierbare Verbindung des Typs 3,3-Diaryl-3H-naphtho[2,1-b]pyran der Formel (I) ist: 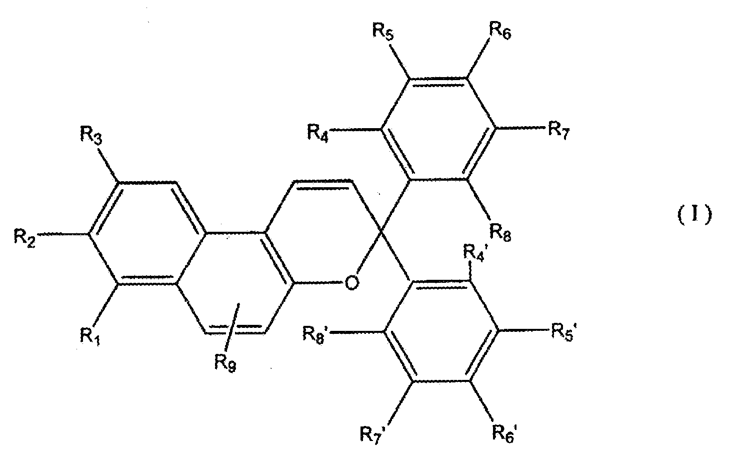 in der
- R₁, R₂ und R₃ so sind, dass:
• ein oder zwei von ihnen eine zweiwertige Gruppe umfassen, die mit einem Monomer (co)polymerisierbar ist, ausgewählt aus der Gruppe, bestehend aus:
• einer Gruppe, die eine Vinyl-Funktion umfasst, ausgewählt aus
-(CₙH₂ₙ)-O-(CH₂)_{n'}-CH=CH₂ und -(CₙH2ₙ)-O-CH=CH₂,
• einer Epoxid-Gruppe,
• einer (Meth)acryloxy-Gruppe, ausgewählt aus
-(CₙH₂ₙ)-OC(=O)CH=CH₂ und -(CₙH₂ₙ)-OC(=O)C(CH₃)=CH₂,
• einer primären Amin-Gruppe,
• einem Anhydrid,
und die andere(n) Gruppe(n) R₁, R₂ und R₃ aus H, (CₙH₂ₙ)-CH₃, (CₙH₂ₙ)-OH ausgewählt sind, n und n' zwischen 0 und 15 liegen,
• oder wenigstens zwei von ihnen wenigstens eine einwertige Gruppe umfassen, die mit einem Monomer (co)polymerisierbar ist, wobei die dritte Gruppe gegebenenfalls aus der Gruppe ausgewählt ist, die aus Wasserstoff, Halogenen und C1- bis C15-Alkylen besteht,
- R₄ bis R₈ und R₄, bis R₈ gleich oder verschieden sind und unabhängig einen Wasserstoff, ein Halogen, ein Hydroxyl, ein C1- bis C15-Alkyl, ein C1- bis C15-Hydroxyalkyl, ein C1- bis C15-Alkoxy darstellen,
- R₉ einen Wasserstoff, ein Hydroxyl, ein C1- bis C15-Alkyl, ein C1- bis C15-Hydroxyalkyl darstellt.

2. Intraokulare Linse gemäß Anspruch 1, **dadurch gekennzeichnet, dass** sie einen optischen Teil und einen haptischen Teil, der aus einem oder mehreren Element(en) besteht, umfasst, wobei wenigstens der optische Teil das pharmazeutisch verträgliche photochrome Polymer umfasst.

3. Intraokulare Linse gemäß einem der Ansprüche 1 oder 2, **dadurch gekennzeichnet, dass** in der Formel (I) R₄ bis R₈ und R₄ bis R₈ gleich oder verschieden sind und unabhängig einen Wasserstoff, ein Methyl, ein Methoxy oder Fluor darstellen und R₉ Wasserstoff ist.

4. Intraokulare Linse gemäß einem der Ansprüche 1 bis 3, **dadurch gekennzeichnet, dass** in der Formel (I) zwei der Gruppen R₁, R₂ und R₃ ein Hydroxyl, eine Gruppe, die Träger einer Carbonsäure ist, ein C1- bis C15-Hydroxyalkyl, ein Isocyanat, ein Epoxid, ein Amino, ein Anhydrid oder eine reaktive Silan-Gruppierung umfassen, wobei diese zwei Gruppen gleich oder verschieden sein können, und die dritte Gruppe ausgewählt ist aus einem Wasserstoff oder einem C1- bis C15-Alkyl, einem Halogen.

5. Intraokulare Linse gemäß einem der Ansprüche 1 bis 3, **dadurch gekennzeichnet, dass** in der Formel (I) die drei Gruppen R₁, R₂ und R₃ unabhängig aus der Gruppe ausgewählt sind, die aus Hydroxylen, Hydroxyalkylen, Isocyanat, einem Anhydrid, Epoxiden, Amino, Gruppen, die Träger einer Carbonsäure sind, und Gruppen, die Träger eines reaktiven Silans sind, besteht.

6. Intraokulare Linse gemäß einem der Ansprüche 4 oder 5, **dadurch gekennzeichnet, dass** wenigstens zwei der Gruppen R₁, R₂ und R₃ aus den Gruppen -(CₙH₂ₙ)-OH, -(CₙH₂ₙ)-NH₂, -(CₙH₂ₙ)-[CH-CH₂-O]-Ring, -(CₙH₂ₙ)-COOH, -(CₙH₂ₙ)-Si(CₘH₂ₘ)₂H und -(CₙH₂ₙ)-Si-CHCH₂, -(CₙH₂ₙ)-Si-(O-CₘH₂ₘ)₃, -(CₙH₂ₙ)-O-C(=O)NH-R₁₀-N=C=O, sind, wobei:
R₁₀ = -(CₙH₂ₙ) oder -(CₙH₂ₙ₋₂) oder (CₙH₂ₙ₋₂)-CH₂-(CₙH₂ₙ₋₂), Aryl oder Aryl-CH₂-aryl,
n zwischen 0 und 15 ist,
m zwischen 1 und 15 ist und
die Aryl-Gruppen 5 bis 20 Kohlenstoffatome
die verbleibenden Gruppen gegebenenfalls H oder ein C1- bis C15-Alkyl umfassen.

7. Intraokulare Linse gemäß Anspruch 6, **dadurch gekennzeichnet, dass** die Gruppen R₁, R₂ und R₃ unabhängig ausgewählt sind aus den Gruppen -(CₙH₂ₙ)-OH, -(CₙH₂ₙ)-NH₂, -(CₙH₂ₙ)-[CH-CH₂-O]-Ring
-(CₙH₂ₙ)-COOH, -(CₙH₂ₙ)-Si(CₘH₂ₘ)₂-H und -(CₙH₂ₙ)-Si-CH=CH₂,
-(CₙH₂ₙ)-Si-(O-CₘH₂ₘ)₃, -OₙH₂ₙ)-O-C=O)NH-R₁₀-N=C=O, wobei:
R₁₀ = -(CₙH₂ₙ) oder -(CₙH₂ₙ-₂) oder (CₙH₂ₙ-₂)-CH₂-(CₙH₂ₙ-₂), Aryl oder Aryl-CH₂-aryl,
n zwischen 0 und 15 ist,
m zwischen 1 und 15 ist und
die Aryl-Gruppen 5 bis 20 Kohlenstoffatome umfassen.

8. Intraokulare Linse gemäß einem der Ansprüche 1 oder 2, **dadurch gekennzeichnet, dass** das photochrome Monomer der Formel: 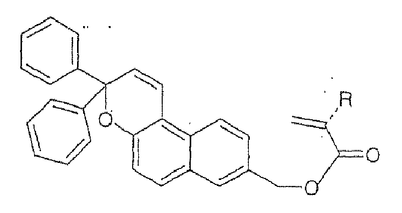 entspricht,
in der R H oder CH₃ darstellt.

9. Intraokulare Linse gemäß einem der Ansprüche 1 bis 8, **dadurch gekennzeichnet, dass** das Polymer außerdem aus wenigstens einem nicht-photochromen Monomer der Formel (III) gebildet wird:
CH₂=C(R₁₁)-COO-(CH₂)ₚ-Y (III)
in der:
* R₁₁ H oder CH₃ ist,
* p 0 bis 10 ist,
* Y ausgewählt ist aus der Gruppe, bestehend aus: Wasserstoff; OH; einer C1-C10-Hydroxyalkyl-Gruppe, einer C1-C10-Fluoralkyl-Gruppe; einer C1-C10-Alkoxy-Gruppe; einer C1-C10-Acyloxy-Gruppe; einer C1-C10-Acylamino-Gruppe; einer C1-C10-Alkylsulfinyl-Gruppe; einer C1-C10-Alkylsulfonyl-Gruppe; einem Rest -(CH2-CH2-O)ₜ H, worin t zwischen 1 und 100 liegt; einem Rest Ar oder W-Ar, wobei Ar einen aromatischen Ring, insbesondere Phenyl, darstellt, der gegebenenfalls mit einer C1-C8-Alkyl-Gruppe, einem Chlor-Atom oder einem Brom-Atom substituiert ist, und W O, S oder NR₁₂ darstellt, worin R₁₂ H oder eine C1-C10-Alkyl-Gruppe ist.

10. Intraokulare Linse gemäß Anspruch 9, **dadurch gekennzeichnet, dass** das Monomer der Formel (III) ausgewählt ist aus der Gruppe, bestehend aus:
einem Alkylmethacrylat, vorzugsweise Methylmethacrylat, einem Hydroxyalkylmethacrylat, einem Hydroxyalkylacrylat, einem Phenoxyalkylacrylat und einem Phenoxyalkylmethacrylat.

11. Intraokulare Linse gemäß Anspruch 10, **dadurch gekennzeichnet, dass** das Monomer der Formel (III) Hydroxyethylmethacrylat oder 2-Phenoxyethylmethacrylat ist.

12. Intraokulare Linse gemäß einem der Ansprüche 1 bis 3, **dadurch gekennzeichnet, dass** das Polymer umfasst:
* ein photochromes Monomer, das aus 3,3-Diphenyl-3H-naphtho[2,1]-pyran-methacrylat, 3,3-Diphenyl-3H-naphtho[2,1]-pyran-acrylat oder einem Gemisch dieser Monomeren besteht, und
* Hydroxyethylmethacrylat oder 2-Phenoxyethylmethacrylat.

13. Intraokulare Linse gemäß einem der Ansprüche 1 bis 12, **dadurch gekennzeichnet, dass** der Verhältnisanteil des photochromen Monomers in dem Polymer zwischen 0,01 % und 5 %, vorzugsweise zwischen 0,02 und 2 % (Massenverhältnisanteil), liegt.
